Europäisches Patentamt

European Patent Office

Office européen des brevets

(19)

(11) **EP 1 519 185 A2**

(12) ## EUROPEAN PATENT APPLICATION

(43) Date of publication:
**30.03.2005 Bulletin 2005/13**

(51) Int Cl.⁷: $G01N\ 3/00$

(21) Application number: **04021730.9**

(22) Date of filing: **08.09.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **24.09.2003 JP 2003332237**

(71) Applicant: **Kansai Technology Licensing Organization Co., Ltd.**
**Kyoto-shi, Kyoto 600-8815 (JP)**

(72) Inventor: **Tsutsumi, Sadami**
**Kyoto-shi Kyoto 616-8105 (JP)**

(74) Representative: **Pfenning, Meinig & Partner**
**Joachimstaler Strasse 10-12**
**10719 Berlin (DE)**

(54) **Method of and device for measuring bulk modulus of minute specimens**

(57)     An object of the present invention is to provide a method of and a device for measuring bulk modulus of a fine specimen.

The present invention has the measurement chamber 6, the reference chamber for comparison 7, the differential pressure measurement means 15 for detecting the pressure difference between both chambers 6, 7, the specimen is contained in the measurement chamber 6, and in a state where both chambers 6, 7 have been set under the standard atmospheric pressure, and in a state where both chambers 6, 7 have been set under the compression atmospheric pressure higher than the standard atmospheric pressure, respectively, the pressure changes are periodically given to both chambers 6, 7, the initial volume of the specimen is detected from the differential pressure signals of both chambers at the time when it is under the standard atmospheric pressure, the compression volume of the specimen is detected from the differential pressure signals of both chambers 6, 7 at the time when it is under the compression atmospheric pressure, and the bulk modulus of the specimen is measured by computing and analyzing these detected signals.

Fig. 1

EP 1 519 185 A2

**Description**

BACKGROUND OF THE INVENTION

1. Field of the Invention

The present invention relates to a method of and a device for measuring the mechanical properties, especially, the bulk modulus of minute living tissues and the other fine specimens.

2. Description of related art

In a cell living culture which aims at performing the regenerative medicine of cartilages, bones and the like, their degrees of maturation is important index for the purpose of determining the timing at which the cell organism is properly implanted into the affected part of the patient, and because of that, it is necessary to precisely measure the strength of these living tissues. However, up until the present, since the shape of a minute and soft living tissue is not prepared and arranged as a test piece for the purpose of precisely measuring the strain and stress, it is difficult to measure the mechanical properties by a usual method of measuring the mechanical properties, and moreover, since it is easily apt to be subjected to the damage of which the tissue is destroyed by the measurement and the like, the mechanical properties could not be measured continually on the way of culturing a cell at stages.

At present, as a known device concerning with the mechanical measurement of the living tissues, there exist devices such as a device in which an organism surface tissue is analyzed and the elasticity, tension, and freshness of skin are measured (for example, see the Patent Document 1), a device in which the beam is irradiated and the degrees of progress of stiff neck, liver cirrhosis, breast cancer or fatty liver are estimated (for example, the Patent Document 2), however, there has not existed such a device in which the hardness of the regenerated tissue itself can be effectively and continually measured on the way of culturing the cell.

[Patent Document 1] Japanese Unexamined Patent Publication No. H11-244266, and

[Patent Document 2] Japanese Unexamined Patent Publication No. H10-000190.

SUMMARY OF THE INVENTION

An object of the present invention is to provide a method of and a device with bulk modulus for precisely measuring the degrees of maturation of living tissues such as objects, especially, cartilage, bone, blood vessel and the like which are not capable of being measured by a usual test device so that these cannot be shaped because these are minute and soft.

The bulk modulus is referred to proportionality constant k in the case where stress P is expressed by the equation $P = -kdV/V$ by Hooke's law at the time when the object of volume V is applied to a certain pressure P and its volume is reduced to dV.

Therefore, an object of the present invention is to provide a method of and a device for firstly measuring the initial volume of the minute specimen under the standard pressure atmosphere, then, measuring the reduced volume of the relevant specimen under the pressurized atmosphere and precisely computing and measuring the bulk modulus of the specimen from the changes of these volumes.

In order to solve the above-described problems, the present invention claimed in claim 1 proposes the basic concept that in a method for detecting the volume change of the specimen at the time when minute specimen such as organism tissue and the like is contained within the measurement chamber having a known volume and the atmospheric.pressure within this measurement chamber has been changed from the first atmospheric pressure to the second atmospheric pressure, the bulk modulus of the specimen is measured by making the volume of the measurement chamber periodically change, measuring the pressure change within the measurement chamber at that time, and computing and analyzing the measurement signal.

The invention claimed in Claims 2 and 5 is a method of and a device for concretely carrying out the above-described basic invention, and in which a measurement chamber, a reference chamber for comparison, and difference pressure measurement means for detecting the pressure difference between the above-described chambers are equipped, a specimen is contained in the measurement chamber, and respectively in the state where both of the measurement chamber and the reference chamber for comparison have been set at the standard atmospheric pressure and in the state where both chambers have been set at an atmospheric pressure higher than the standard atmospheric pressure, the invention is a method and a device for carrying out in which the bulk modulus of the specimen is measured by giving small periodical volume changes to both chambers, by detecting the initial volume of the specimen from the difference pressure signal of the both chambers at the time when it is under the standard atmospheric pressure, by detecting the compressed volume of the specimen from the difference pressure signals of both chambers at the time when these are under compressed atmospheric pressures, and by computing and analyzing these detected signals.

The invention claimed in Claim 3 and in the following Claims, is to provide a computing equation for the purpose of computing the bulk modulus using the above-described method and device and besides that, is to provide effective concrete means for the purpose of carrying out the present invention.

According to the present invention, any shape is available if the specimen is a minute object which is capable of being inserted into the sample entering portion of the measurement chamber, therefore, the measurement of the minute living tissue remained as it lives can be done. Conventionally, since the strength of the cell as it lives in an indeterminate form is not capable of being measured by an ordinal stress or strain meter or a strain gauge and the like, the measurement has been solely done using the samples properly dealt with as an index. However, according to the present invention, since even if it is a living tissue having an indeterminate form, it can be extremely precisely measured as described above, for example, when the bulk modulus of living cartilage of the healthy person and the bulk modulus of the living cartilage made for therapy are compared, the degree of maturation of the living cartilage for its therapy is capable of being measured as it is.

Specifically, according to the present invention, an organism tissue in an indeterminate form is capable of being quantitatively measured as it is from the viewpoints of its mechanical properties, for example, the degree of distortion, modulus and the like, and it contributes to researches in the fields of the regeneration medicine, bioengineering and the like in an epoch-making way.

BRIEF DESCRIPTION OF THE DRAWING

For a more complete understanding of the present invention and the advantages thereof, reference is now made to the following detailed description taken in conjunction with the accompanying drawings, in which:

Fig.1 is a longitudinal cross-sectional view showing one example of a device carrying out a method according to the present invention;
Fig.2 is a cross-sectional view taken on line A-A showing a piston central axis in the direction of arrows of Fig.1;
Fig.3 is a schematic diagram showing a measurement circuit constitution which takes out the difference of the pressures detected from the respective chambers within the housing and deals with that; and
Fig.4 is a diagram prepared by plotting output coordinates at the time when steel balls numbered from 0 piece to 4 pieces were contained in the measurement chamber and measured, showing the straight line A in the case where the housing was placed under the ambient pressure (0 kPa), the straight line B in the case where the housing was placed under the atmospheric pressure of 50 kPa, and the straight line C in the case where the housing was placed under the atmospheric pressure of 100 kPa.

DESCRIPTION OF THE PREFERRED EMBODIMENTS

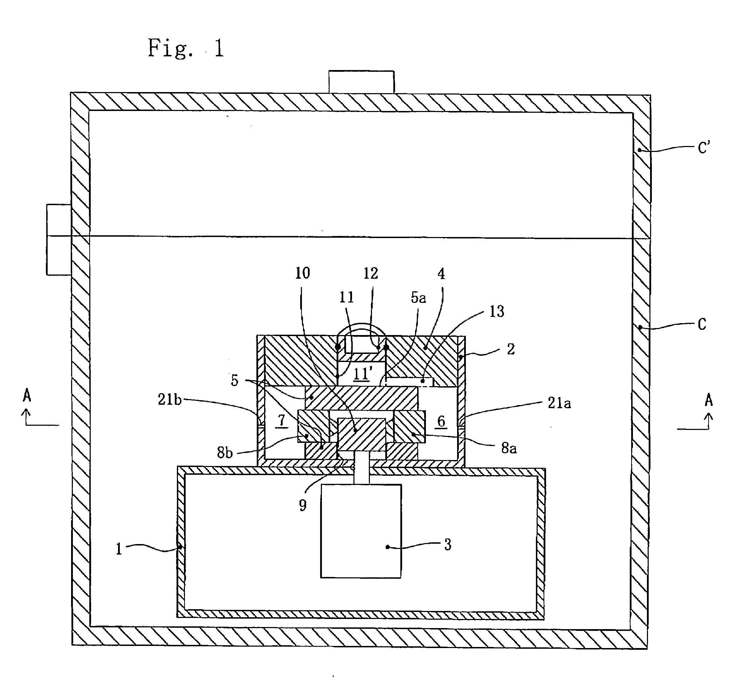

Fig.1 is a vertical section showing one example of a device carrying out the present invention, the reference numeral 1 denotes a box at base composed of hard plastic and the like, the reference numeral 2 denotes a housing for containing a measurement chamber supported on the upper end of the box at base 1, the reference numeral 3 denotes a motor for rotating and driving a piston drive cam within the housing 2, and the reference character C denotes an outer sealing housing which is equipped with a lid C'.

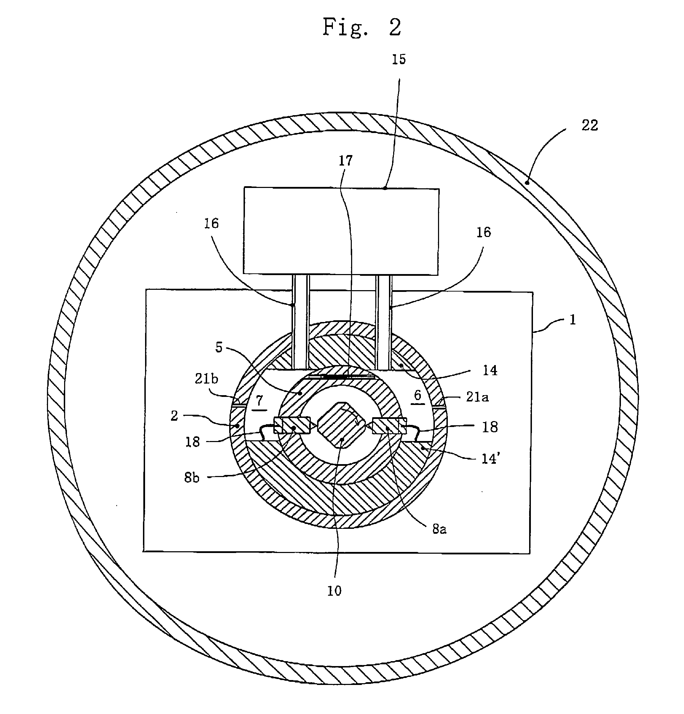

In this case, the housing 2 is in a cylindrical shape, the interior space below a top plate 4 is partitioned into a measurement chamber 6 and a reference chamber for comparison 7 by a plate block 5 and a wall block 14 and the like described later concerning with Fig.2. In the plate block 5, a cylinder chamber for containing a pair of pistons in the same shape comprising a piston 8a which is coming in and going out from the measurement chamber 6 and a piston 8b which is coming in and going out from a reference chamber for comparison 7, are equipped, and intermediate lower portion of the cylinder chamber communicates with a cam axis bore 9 as similarly formed in the plate block 5. The piston drive cam 10 supported on the output axis of the motor 3 is fitted into the above-described cam axis bore 9.

The top plate 4 has a opening 11, a cap 12 is fitted into the opening 11. A measuring specimen is putted into the upper surface of the plate block 5 or a sample entering portion 11' by removing the cap 12. Since the sample entering portion 11' is in a condition completely communicated with the measurement chamber 6 via the flow channel 13 formed on the lower surface of the top plate 4, it forms a space as an extension portion of the measurement chamber 6. The reference numerals 21a, 21b are communication channel which is communicated with the measurement chamber 6, the volume chamber for comparison 7 and the outer housing C, the atmospheric pressure within the volume chamber for the measurement chamber 6 and comparison 7 are kept in the same atmospheric pressure with the housing C, owing to this, however, since it is extremely narrow flow channel, the flow channel resistance is designed in a higher state.

Fig.2 is a cross-sectional view taken on line A-A showing the piston central axis of Fig.1. As it is clear from Fig.2, the wall blocks 14, 14' formed within the back surface and front surface arc range of the housing 2 in a cylindrical shape determines the size of the measurement chamber 6 and the reference chamber for comparison 7 as well as fixes the plate block 5. Conducting pipes for detection 16, 16 penetrates through the wall block 14, achieving to the a pair of interior detection ends (not shown) of the differential pressure gauge 15 entering from the outside of the housing 2, and open the openings on the measurement chamber 6 and the reference chamber for comparison 7, respectively. As an element structure of a pressure sensor constituting the differential pressure gauge 15, a semiconductor diaphragm type, a piezoelectric type, an electrostatic capacity type, an elastic diaphragm type and the like can be used, however, in this mode for carrying out, an electrostatic capacity type which is easily structured as a differential pressure gauge has been employed. In such an electrostatic capacity type differential pressure gauge sensor, the above-described pair of interior detection ends are referred to both surfaces of the silicon diaphragm which has supported the central electrode located between the upper and lower fixed electrodes, and the electrostatic capacity between the upper and lower respective electrodes and the central electrode follows the positional displacement of the silicon diaphragm which has corresponded to the differential pressure. It should be noted that the reference numeral 17 is a microtube channel which is communicated with the measurement chamber 6 and the volume chamber for comparison 7, the microtube channel has been provided for the purpose of making both chambers 6, 7 in a pressure balanced state at a sufficient time constant, therefore, originally, it might be not particularly provided.

In the same way, in Fig.2, the reference numeral 18 denotes a return spring 18 which supports the rear ends of the pistons 8a and 8b, always pushes the tip of the piston to the cam 10, insures the reciprocating motion of the piston according to the cam rotation. It should be noted that it is desirable that the microtube communication channels 21a, 21b communicating with respective chambers of the measurement chamber 6 and the reference chamber for comparison 7 and the exterior sealing housing C are provided in both chambers 6, 7, however, even in the case where the communication channel has been provided only in any one of these chambers, the other chamber is also capable of being made into the same pressure after a certain time passes through the microcommunication channel 17 between both chambers 6, 7.

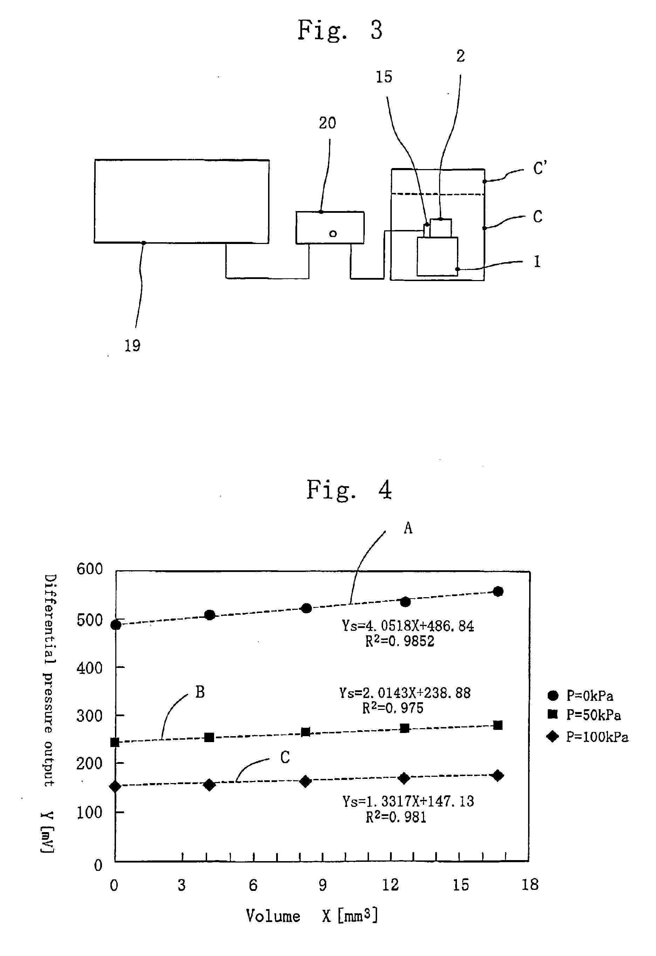

Fig.3 is a schematic diagram showing a measurement circuit constitution for taking out the differential pressure detected from the housing 2 and dealing with that. The differential pressure output of the differential pressure gauge 15 is supplied to a FFT analyzer (Fast Fourier Transform device) 19, owing to this FFT analyzer 19, it is outputted as a precise differential pressure signal which has removed the noise and the like occurring accompanying with the piston motion and the like, the bulk modulus of the specimen is calculated by the computing circuit embedded in it. It should be noted that a computing circuit and an outputting device using only for that purpose might be further connected to the exterior of the FFT analyzer 19 and the like. Noted that the reference numeral 20 denotes an electrical source for the differential pressure gauge 15, the FFT analyzer 19 and the like. The measurement principle in the case where the device constitution as described above has been used is as the followings. The entire of the volumenometer (1, 2, 15) is enclosed with the sealing housing C.

First, it is supposed that the volume of the measurement chamber 6 and the reference chamber for comparison 7 set under the standard atmospheric pressure (for example, ambient pressure) is changed by $\Delta V$ periodically at the equiphase by the pistons 8a, 8b coming in and going out from the chamber. Here, it is also supposed that the volume and the pressure of the volume chamber for comparison 7 of the measurement chamber 6 including the sample entering portion 11' are V1, P1, and the volume and the pressure of the volume chamber for comparison 7 are V2, P2, respectively. Since the microtube channel 17 between both chambers 6, 7 is set so that the equation of P1 = P2 is held by a time constant (about 1 minute) which is sufficiently long with regard to the change of $\Delta V$, if it is considered that the change of $\Delta V$ occurs at a certain periodical cycle, the average pressure within the periodical cycle can be expressed as the equation of $\overline{P1} = \overline{P2} = P$. In this state, supposed that polytropic index which gives the change to PV is n, in the quasi-static process,

$$\cdot\ P1V1^{n} = Const \tag{1}$$

Both sides are differentiated by time,

$$\cdot\ P1nV1^{n-1}\ dV1 + dP1V1^{n} = 0 \tag{2}$$

From the above-expressed equation, when the volume increment dV1 and the pressure increment dP1 in this differential equation (2) are substituted by the volume change $\Delta V1 = \Delta V$ and corresponding pressure change $\Delta P1$, the resolution of the pressure instant value P1 of the measurement chamber is also counted per $\Delta V$ generation periodical cycle, and naturally, the equation of [P1] = P is applied. Therefore, as the pressure change equation,

$$\cdot \Delta P1 = -nP\Delta V / V1 \qquad (3)$$

the above-described equation (3) is obtained, similarly, also with regard to the reference chamber for comparison, the equation of $\Delta V2 = \Delta V$ is held,

$$\cdot \Delta P2 = -nP\Delta V / V2 \qquad (4)$$

The above-described equation (4) is obtained, and in the state in which the sample is not contained within the measurement chamber (sample entering portion 11'), and it is expressed as the following equation:

$$\cdot \Delta P1 - \Delta P2 = nP\Delta V (1/V1 - 1/V2) \qquad (5)$$

Here, it is supposed that the sample having the minute volume X (on the order of 1 / several hundreds of V1 and 1/1000) is contained in the measurement chamber (the sample entering portion 11') and the remaining volume is Vx, the differential pressure in accordance with the volume occurs between the measurement chamber 6 and the reference chamber for comparison 7. The relationship is expressed by the equation (6).

$$\bullet \ \Delta P1 - \Delta P2 = nP\Delta V[1/(V1-X) - 1/V2]$$
$$= nP\Delta V (1/Vx - 1/V2) \qquad (6)$$

As described above, since the reason why the equation of [P1] = [P2] = P could be employed in the above-described equation (6) is that the air resistance between the measurement chamber 6 and the reference chamber for comparison 7 is moderately large on the order that the pressures of both chambers are balanced with regard to a time constant (about one minute) which is sufficiently long, such an air communication relationship is also held between the respective chambers 6, 7 and the exterior space of the volumenometer, and the volumenometer exterior pressure as a known or set value of the volumenometer can be used for this P value. Owing to this, if the differential pressure $\Delta P1 - \Delta P2$ is measured, since in the right side of the equation (6), all but except the volume Vx are known constant, this Vx, that is, the sample volume X therefore can be easily determined.

Furthermore, the volume V1 of the measurement chamber 6 including the sample entering portion 11' and the volume V2 of the reference chamber for comparison 7 have been previously set so that the it is held as V1 = V2 = V, the first line of the equation (6) is expressed as follows:

$$\cdot \Delta P1 - \Delta P2 = nP\Delta V [1/(V-X) - 1/V] \qquad (7)$$

when this right side is changed to equivalent fractions with a common denominator,

$$\bullet \ \Delta P1 - \Delta P2 = nP\Delta V [(V-V + X) / V(V-X)]$$
$$= [nP\Delta V / (V-X)]X/V$$
$$= [nP\Delta V(V+X) / (V^2-X^2)]X/V \qquad (8)$$

The above-described equation (8) is obtained.

And then, since square $X^2$ of the minute volume of the denominator can be ignored with regard to $V^2$, the following equation is obtained.

$$\bullet \ \Delta P1 - \Delta P2 = [nP\Delta V [ (V+X) / V^2]X/V$$
$$= nP\Delta V (X/V^2 + X^2/V^3) \qquad (9)$$

Similarly, the second term of the right side is ignored, and the following equation is obtained.

$$\bullet \; \Delta P1 - \Delta P2 = (nP\Delta V/V^2)X \qquad (10)$$

And then, the above-described equation (10) can be used for the purpose of easily determining sample volume X.

Next, a method in which the effective output Ys repressing the actual differential pressure $\Delta P2 - \Delta P1$ is extracted from the output signal of the differential pressure gauge will be described below. The output signal of the differential pressure $\Delta P1 - \Delta P2$ was generated by the fact that the volume of the specimen X was injected in the measurement chamber 6 is after all, the vibration component of the frequency corresponding to the periodical cycle movement of the pistons 8a, 8b generating the volume change $\Delta V$.

Here, if the motions of the pistons 8a, 8b are simple harmonic motions expressed as a sine wave at the time axis, the major component of the output of the differential pressure gauge is a signal of the sine wave by the vibration frequency, however, as it is clear from Fig.2, the piston drive cam 10 for driving the pistons 8a, 8b against the spring 18 may be referred to that in a shape of deformed octagon that the vertexes of square were cut, largely, it comprises a basic wave component (primary component) that the distance between the vertexes of the square is made as one periodical cycle and double frequency (secondary component) that the distance between the vertexes having the obtuse angles of the deformed octagon is made one periodical cycle, and others, the noises due to the vibration and bombardment of the device are also added. Upon the carrying out the present invention, in the case where 76 Hz which is four times (value determined by multiplying the four sides of the square) of the number of revolutions of the pseudo-square cam whose vertexes were cut of 19 Hz of the square cam is made frequency of the primary component, it could be most precisely measured.

Therefore, in the mode for carrying out of the present invention, as shown in Fig.3, the output of the differential pressure gauge 15 is inputted into a FFT analyzer 19, where the basic wave component (primary component) showing the pressure variation and double frequency component (secondary component) are extracted out of the signals obtained by a high speed Fourier conversion, the third harmonic wave or more is ignored as a noise. In this case, the secondary component is a signal generated by the deformed octagon, it exists as a signal component which should be subtracted from the error portion generated by four vertexes deformation including in the primary component. Because of it, the differential pressure gauge processed by the FFT computing processing is used as the value of $Ys \propto \Delta P1 - \Delta P2$ that the secondary component has been subtracted from the primary component. In this way, from $\Delta P1 - \Delta P2 = nP\Delta V(1/Vx - 1/V2)$ of the equation (6), the following equation is obtained:

$$\bullet \; X = V1 - Vx \qquad (11)$$

And then, the volume of the specimen X under the standard pressure P (for example, ambient pressure) is obtained by the above-described equation (11) or simply the volume of the specimen X under the standard pressure P (for example, ambient pressure) is obtained from $\Delta P1 - \Delta P2 = (nP\Delta V/V^2)X$ of the equation (10).

Next, the volume X' of the same specimen which has been changed (contracted) under the pressure P' that has been higher pressure to which similar volume change $\Delta V$ is applied in the case where the volumenometer itself is placed under an atmospheric pressure P' higher than the standard pressure P is obtained.

$$\bullet \; X' = V1 - Vx' \qquad (12)$$

From the values of these sample volume X and X', the volume strain $\varepsilon v$ and the bulk modulus K can be calculated by the equations listed as follows:

$$\bullet \; \varepsilon v = (X - X')/X \qquad (13)$$

and

$$\bullet \; K = (P' - P)/\varepsilon v \qquad (14).$$

Example 1

A regression line for Ys-X calibration for the purpose of determining volume X is prepared from the differential pressure output Ys with regard to unknown sample. For this purpose, here, first, the output is determined at the time when for example, a steel ball was inputted into the sample entering portion 11' of the measurement chamber under the ambient pressure (0 kPa when expressed in the unit of kPa). At this time, it is supposed that the volume of one piece of the steel ball is x, and in the case where the number of pieces is N, it is supposed that the equation of X = Nx is held. The actual volume of the steel ball, x is 4.19 mm$^3$, the regression line A of Fig.4 is a line that output coordinates from 0 piece to 4 pieces of this steel balls were plotted and was prepared. Next, the volumenometer itself is contained in the sealing container and the compression air is sent, similarly, the outputs of the volumenometer are determined with regard to the number of samples and the graphical representation is prepared. It is assumed that the bulk modulus of the steel ball is sufficiently large, and the volume change hardly occurs with respect to the pressure (high pressure) which is applied. It should be noted that the regression line B in the measurement under a high pressure shows the results in the case where the pressure is 50 kPa, and the regression line C shows the results in the case where the pressure is 100 kPa.

These regression lines have the respective eigenvalues at X coordinate, 0 (without sample), and these are primary lines having a certain gradient, and it is indicated that the general expression at the time when it is under the ambient pressure is expressed as follows:

$$• \ Ys = aX + b \tag{15}$$

And, it is indicated that the general expression at the time when it is under the high pressure is expressed as follows:

$$• \ Ys' = a'X' + b' \tag{16}$$

The equations of the straight lines actually calculated using a numerical calculation method were as the followings:

$$Ys = 4.0518X + 486.84 \ [mV],$$

in the case of A (under standard pressure: ambient pressure),

$$Ys' = 2.0413X' + 238.88 \ [mV],$$

in the case of B (under 50 kPa), and

$$Ys' = 1.3317X' + 147.13 \ [mV],$$

in the case of C (under 100 kPa).

In the preparation of graphical representation, the contributions $R^2$ showing the precision of the straight line relationship with regard to Ys, Ys', and X, X' are 0.9852, 0.975, and 0.981, respectively, and it is clear that any of these has a high precision.

Therefore, it is needless to say that the sample volumes X and X' at the time when it is under the standard pressure, and at the time when it is under a high pressure or at the time when it is under the compression are capable of being calculated by applying ($\Delta P2-\Delta P1$) and ($\Delta P2'-\Delta P1'$) which were obtained from the output Ys and Ys' of the FFT analyzer to the above-described equations (1) and (2), and it is possible that these are immediately determined from the positions (or numerical values within the table whose addresses are Ys and Ys') on the straight lines of the above-described equations of Ys = aX + b and Ys' = a' X' + b', and as a device for carrying out a method of the present invention, it is easier that the device is initially set as a method employing the later Ys method and Ys' method.

Example 2

The volume of silicone rubber particle whose mechanical properties are known and which is similar to a living cartilage tissue was measured using the above-described calibration curves. First, in Table 1, the known mechanical properties are shown as well as the mechanical properties of the steel ball.

Table 1

| Kind of samples | Volume V [mm$^3$] | Young' modulus of elasticity E [kPa] | Volume elasticity K [kPa] |
|---|---|---|---|
| Steel ball | 4.19 | 200 × 106 | Extremely high value (about 170 × 106) |
| Silicone rubber | 11.5 | 80 | 670* |

* Where, supposed that K = E / 3 (1-2ν) and Poison's ratio ν is 0.48.

Next, the measurement results obtained by applying the FFT processed output of the differential pressure gauge to the regression line for calibration are shown in Table 2.

Table 2

| Pressure P [kPa] | FFT processed output of differential pressure gauge [mV] / 76 Hz - 152 Hz | Volume (calculated value) [mm$^3$] |
|---|---|---|
| 0 | 529.431 | 10.5 (- 1.0*) |
| 100 | 159.084 | 8.98 |

* showing that the error with respect to the actual volume is -1.0.

When the volume elasticity K of the silicone rubber particle is calculated using the volume obtained by the above-described measurement, it is determined by the equation of K = P / εv, that is to say, K = 100 / [(10.5-8.98) / 10.5] = 690 [kPa]. This value is only in the error range of + 3% or less with respect to the known bulk modulus K = 670 as one of the known mechanical properties listed in Table 1, and moreover, since it cannot be said that this K value is the absolute true value with respect to the same silicone rubber particle, it can be interpreted that approximately precise value was obtained.

Although a piston of cam drive / spring using a method of returning was employed as volume change giving means for the purpose of giving the change of ΔV with respect to the measurement chamber and reference chamber for comparison, the other mechanical displacement means such as a piston using an electromagnetic drive method, a diaphragm, means using a diaphragm method, and the like may be employed. Moreover, an inert gas may be used as a gas filling the interior and exterior of the measurement chamber and the volume chamber for comparison and as a result, it is possible to prevent the oxidation and deterioration of the device structure and sample. Furthermore, referring to the atmospheric pressure within the outer sealing housing C, if first, it is changeably set for 3 stages in such a way that it is set at one atmospheric pressure, and then, for example, 1.3 atmospheric pressure, and 1.5 atmospheric pressure, and the differential pressure outputs at the respective stages are measured and calculated, more precise regression lines are capable of being prepared, therefore, the volume elasticity of the specimen can be determined with a higher precision.

As it is clear from the above-described Embodiments, a method of the present invention can quickly and precisely measure the volume elasticity which is one of the mechanical properties with respect to the variety of specimens whose shapes are not specified, and can measure it as it lives if the specimen is a minute living tissue. Therefore, the present invention can be utilized as a measuring gauge for medical use for the purpose of measuring the degree of maturation of a living tissue such as cartilage, bone, blood vessel and the like which were cultured, and it can be also utilized for the purpose of elucidating the mechanical properties and mechanical characteristics of the wide range of substances such as uncertain soft substances, for example, synthetic fibers, plant fibers, seeds, fat and the like.

It will also be appreciated that, although a limited number embodiments of the invention have been described in detail for purposes of illustration, various modifications may be made without departing from the spirit and scope of the invention. Accordingly, the invention should not be limited except as by the appended claims.

**Claims**

**1.** In a method in which a minute specimen such as a living tissue is contained in a measurement chamber having known volume, and a volume change of said specimen is detected when the atmospheric pressure within this measurement chamber was forced to change from first atmospheric pressure to second atmospheric pressure ,

said method of measuring a volume bulk modulus of a fine specimen, wherein volume within said measurement chamber is periodically changed, the pressure variation within said measurement chamber at that time is measured, and bulk modulus of said specimen is measured by computing and analyzing its measurement signal.

2. In a state where a measurement chamber, a reference chamber for comparison and differential pressure measurement means for detecting a pressure difference between said both chambers are equipped, a specimen is contained in said measurement chamber, and both said measurement chamber and said reference chamber for comparison have been set under the standard atmospheric pressure, and in a state where said both chambers have been set under a compression atmospheric pressure higher than the standard atmospheric pressure, respectively, a method of measuring bulk modulus of a minute specimen, wherein said both chambers are periodically given a pressure change, the initial volume of said specimen is detected from a differential signal of said both chambers when it is under the standard atmospheric pressure, a compression volume of said specimen is detected from a differential pressure signal of said both chambers when it is under a compression atmospheric pressure and a bulk modulus of said specimen is measured by computing and analyzing these detected signals.

3. The method of measuring a bulk modulus as claimed in Claim 2, wherein it is supposed that the relevant volume of a measurement chamber is V1, the relevant volume of a reference chamber for comparison is V2, the same volume change at the equiphase giving to both chambers is $\Delta V$, the initial volume of said specimen contained under said standard atmospheric pressure is X, the relevant volume within said measurement chamber except for the volume of its specimen is Vx, changes of pressure P1, P2 within the respective chambers of said measurement chamber and said reference chamber for comparison are $\Delta P1$, $\Delta P2$, the average of the same P1, P2 is [P1] = [P2] = P,

the compression volume of said specimen contained under said compression atmospheric pressure is X', the relevant volume within said measurement chamber except for its volume of said specimen is Vx', changes of pressure P1', P2' within the respective chambers of said measurement chamber and said reference chamber for comparison are $\Delta P1'$, $\Delta P2'$, the average of the same P1', P2' is [P1'] = [P2'] = P',

volume increment dV and pressure increment dP in differentiation equation of $PV^n$ polytropic change which are in common with said respective chambers are substituted by said volume change $\Delta V$, pressure changes $\Delta P1$, $\Delta P2$ and $\Delta P1'$, $\Delta P2'$, respectively, following differential pressure approximate equations at both times are obtained:

$$\bullet\Delta P1 - \Delta P2 = nP\Delta V (1/Vx - 1/V2) \tag{1}$$

$$\bullet\Delta P1' - \Delta P2' = nP'\Delta V (1/Vx' - 1/V2) \tag{2}$$

and,

said initial volume of said specimen X corresponding to volume Vx placed under the standard atmospheric pressure and said compression volume of said specimen X' corresponding to volume Vx' placed under the compression atmospheric pressure are determined from $\Delta P1 - \Delta P2$, $\Delta P1' - \Delta P2'$ detected by said differential pressure gauges and said (1), (2) equations.

4. The method of measuring bulk modulus as claimed in Claim 2 or 3, wherein said standard atmospheric pressure is made ambient pressure.

5. A device for measuring bulk modulus of a fine specimen, wherein said device has a measurement chamber for containing said specimen, a reference chamber for comparison, volume change giving means for periodically giving the same volume change to said both chambers, means for exchanging atmospheric pressure within said both chambers into at least two stages of first pressure and second pressure, and means for detecting a pressure difference within said both chambers when said volume change was given, and bulk modulus of said specimen is calculated by measuring compression volume of said specimen from the relevant differential pressure of both chambers.

6. The device for measuring bulk modulus as claimed in Claim 5, wherein a structure body comprising said measurement chamber, said reference chamber for comparison, said volume change giving means, and differential measurement means for measuring differential pressure is contained within a sealing housing capable of introducing a pressurized gas, said measurement chamber and said reference chamber for comparison are communicated within said sealing housing via a flow channel having a high communication resistance.

7. The device for measuring bulk modulus as claimed in Claim 5 or 6, wherein said volume change giving means comprises a pair of pistons which come in and go out from said measurement chamber and said volume chamber for comparison.

8. The device for measuring bulk modulus as claimed in any one of Claims 5 - 7, wherein said volume change giving means is a diaphragm.

9. The device for measuring bulk modulus as claimed in any one of Claims 5 - 8, wherein it was made so that atmospheric pressures of said measurement chamber and reference chamber for comparison are set at a plurality of different atmospheric pressures.

10. The device for measuring bulk modulus as claimed in any one of Claims 5 - 9, wherein said device has means for computing bulk modulus by performing Fourier conversion to and analyzing an output of said differential pressure measurement means.

Fig. 1

EP 1 519 185 A2

Fig. 2

# Fig. 3

# Fig. 4

$Ys=4.0518X+486.84$
$R^2=0.9852$

$Ys=2.0143X+238.88$
$R^2=0.975$

$Ys=1.3317X+147.13$
$R^2=0.981$

● P=0kPa
■ P=50kPa
◆ P=100kPa

Differential pressure output  Y [mV]

Volume  X [mm³]